# EUROPEAN PATENT APPLICATION

(11) **EP 4 647 012 A1**
(43) Date of publication of application: **12.11.2025**
(21) Application number: 25172277.3
(22) Date of filing: 24.04.2025
(51) Int. Cl.: A61B 6/00

(54) **DETECTOR IDENTIFICATION APPARATUS AND MEDICAL IMAGING SYSTEM**

(30) Priority: 09.05.2024 CN 202421000538 U
(71) Applicant: GE Precision Healthcare LLC, Waukesha, WI 53188 (US)
(72) Inventor: LIU, Jie, Waukesha, 53188 (US); MA, Li, Waukesha, 53188 (US); DU, Pengfei, Waukesha, 53188 (US); LIU, Hui, Waukesha, 53188 (US)
(74) Representative: Kilburn & Strode LLP

(57) **Abstract**

Provided in embodiments of the present application are a detector identification apparatus and a medical imaging system. The detector identification apparatus includes: a marking module associated with a detector and including at least one magnetic block; and an identification module that senses the magnetic block and generates marking information of the detector according to a sensing result.

## Description

### TECHNICAL FIELD

Embodiments of the present application relate to the technical field of medical imaging, in particular to a detector identification apparatus and a medical imaging system.

### BACKGROUND

In a medical imaging system, radiation from a radiation source is emitted toward a subject under examination. A portion of the radiation, after passing through the subject under examination, is received by a detector, and output information is generated based on the amount or intensity of the radiation received by the detector. The output information is processed correlatively to generate a displayable medical image for inspection.

### SUMMARY

The inventors have found that: a medical imaging system may comprise more than one detector. For configurations with more than one detector, there are situations in which the system cannot acquire marking information of the detectors. For example, the detectors are wireless detectors without marking information for communication with the system. In this case, the system cannot identify or distinguish the detectors mounted at different positions, and users need to visually identify the detectors, which easily leads to the complication of an operation process and erroneous operations.

With respect to at least one of the above technical problems, embodiments of the present application provide a detector identification apparatus and a medical imaging system.

According to one aspect of the embodiments of the present application, a detector identification apparatus is provided. The apparatus comprises: a marking module associated with a detector and comprising at least one magnetic block; and an identification module, the identification module sensing the magnetic block and generating marking information of the detector according to a sensing result.

According to another aspect of the embodiments of the present application, the marking module is disposed on the detector, or the marking module is disposed on a support assembly supporting the detector.

According to another aspect of the embodiments of the present application, the magnetic block is disposed on the detector in a preset pattern comprising at least one of number information, position information, or size information of the magnetic block.

According to another aspect of the embodiments of the present application, the preset patterns of the magnetic blocks disposed on different detectors are different.

According to another aspect of the embodiments of the present application, the marking module is connected to a housing of the detector; and the identification module is connected to a support assembly supporting the detector.

According to another aspect of the embodiments of the present application, the marking module further comprises: an accommodating assembly configured to accommodate the magnetic block, the accommodating assembly being connected to a housing of the detector.

According to another aspect of the embodiments of the present application, the accommodating assembly comprises: a positioning member connected to the housing of the detector and having a positioning hole formed on a side surface away from the housing of the detector, the positioning hole being configured to accommodate the magnetic block; and a cover plate connected to the positioning member in such a manner as to clamp the magnetic block.

According to another aspect of the embodiments of the present application, the identification module comprises: at least one magnetic sensor disposed opposite to the magnetic block, the distance between the magnetic sensor and the magnetic block being less than a first preset distance.

According to another aspect of the embodiments of the present application, the identification module further comprises: a mounting assembly connected to the magnetic sensor and a support assembly supporting the detector, and mounting the magnetic sensor on the support assembly in such a manner as to float in a first direction, the first direction being a direction of the magnetic sensor opposite to the magnetic block.

According to another aspect of the embodiments of the present application, the mounting assembly comprises: a body member connected to the magnetic sensor; a guide member, a first end of the guide member being connected to the support assembly, and the body member driving the magnetic sensor to move along the guide member; and an elastic member, a first end of the elastic member being connected to the body member, and a second end of the elastic member being connected to the support assembly or the guide member.

According to another aspect of the embodiments of the present application, the body member has a hole portion formed therein, at least a portion of the guide member being located in the hole portion, and the guide member has a limiting portion formed at a second end thereof, and when the body member is located at a first position, an end surface of the limiting portion facing the support assembly abuts against an end surface of the body member facing the detector.

According to another aspect of the embodiment of the present application, the guide member is located at a position coinciding with a central axis of the body member, a plurality of elastic members are provided, and the elastic members are located at positions symmetrical relative to the body member.

According to another aspect of the embodiments of the present application, the mounting assembly further comprises: a blocking member connected to the body member, an end surface of the blocking member facing the detector being located at a position flush with an end surface of the magnetic sensor facing the detector, or an end surface of the blocking member facing the detector being located at a position closer to the detector than an end surface of the magnetic sensor facing the detector.

According to one aspect of the embodiments of the present application, a medical imaging system is provided. The system comprises: a detector, the detector identification apparatus according to any one of the previous aspects; and a controller, the controller, according to marking information of the detector generated by the detector identification apparatus, generating indication information related to the detector.

According to another aspect of the embodiments of the present application, the system further comprises: a display, the display displaying position information of the detector according to the indication information.

With reference to the following description and drawings, specific implementations of the embodiments of the present application are disclosed in detail, and the way in which the principles of the embodiments of the present application can be employed are illustrated. It should be understood that the embodiments of the present application are not limited in scope thereby. Within the scope of the spirit and clauses of the appended claims, the embodiments of the present application include many changes, modifications, and equivalents.

### BRIEF DESCRIPTION OF THE DRAWINGS

The included drawings are used to provide further understanding of the embodiments of the present application, which constitute a part of the description and are used to illustrate the implementations of the present application and explain the principles of the present application together with textual description. Evidently, the drawings in the following description are merely some embodiments of the present application, and those of ordinary skill in the art may obtain other implementations according to the drawings without involving inventive effort. In the drawings:
FIG. 1 is a schematic diagram of a medical imaging system according to embodiments of the present application;
FIG. 2 is a schematic diagram of a detector identification apparatus according to embodiments of the present application;
FIGS. 3 and 4 are schematic diagrams of a preset pattern according to embodiments of the present application;
FIG. 5 is a schematic diagram of a marking module according to embodiments of the present application;
FIG. 6 is a schematic diagram of a magnetic sensor according to embodiments of the present application;
FIG. 7 is a schematic diagram of an identification module according to embodiments of the present application; and
FIGS. 8 and 9 are additional schematic diagrams of medical imaging systems according to embodiments of the present application.

### DETAILED DESCRIPTION

The foregoing and other features of the embodiments of the present application will become apparent from the following description with reference to the drawings. In the description and drawings, specific implementations of the present application are disclosed in detail, and part of the implementations in which the principles of the embodiments of the present application may be employed are indicated. It should be understood that the present application is not limited to the described implementations. On the contrary, the embodiments of the present application include all modifications, variations, and equivalents which fall within the scope of the appended claims.

In the embodiments of the present application, the terms "first", "second", etc., are used to distinguish different elements, but do not represent a spatial arrangement or temporal order, etc., of these elements, and these elements should not be limited by these terms. The term "and/or" includes any and all combinations of one or more associated listed terms. The terms "comprise", "include", "have", etc., refer to the presence of described features, elements, components, or assemblies, but do not exclude the presence or addition of one or more other features, elements, components, or assemblies. Similar terms such as "connect", "link", "couple" used in the embodiments of the present application are not limited to physical or mechanical connections, but may include electrical connections, whether direct or indirect.

In the embodiments of the present application, the singular forms "a" and "the" include the plural forms, and should be broadly construed as "a type of" or "a class of" rather than being limited to the meaning of "one". Furthermore, the term "the" should be construed as including both the singular and plural forms, unless otherwise specified in the context. In addition, the term "according to" should be construed as "at least in part according to ..." and the term "based on" should be construed as "based at least in part on ...", unless otherwise specified in the context.

The features described and/or illustrated for one implementation may be used in one or more other implementations in the same or similar way, be combined with features in other embodiments, or replace features in other implementations. The term "include/comprise" when used herein refers to the presence of features, integrated components, steps, or assemblies, but does not preclude the presence or addition of one or more other features, integrated components, steps, or assemblies.

In the embodiments of the present application, an exemplary description is performed for a detector identification apparatus and a medical imaging system of the present application by using an X-ray imaging scenario as an example. Those skilled in the art will understand that the present application is also applicable to other radiographic imaging systems or imaging systems based on other high-frequency electromagnetic energy.

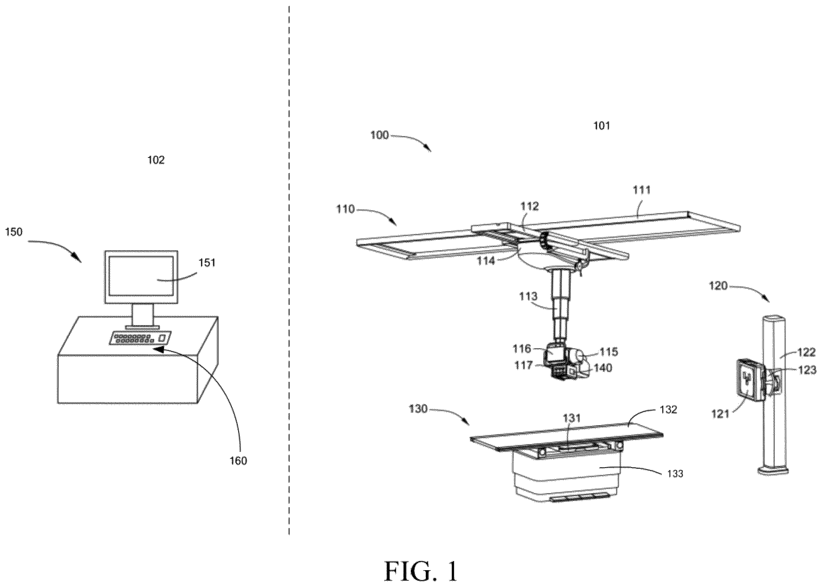

FIG. 1 is a schematic diagram of a medical imaging system according to embodiments of the present application. As shown in FIG. 1, the medical imaging system 100 includes a suspension apparatus 110, a wall stand apparatus 120, and an examination table apparatus 130 arranged in a scanning room 101, as well as a control apparatus 150 arranged in a control room 102. The suspension apparatus 110 includes a longitudinal guide rail 111, a transverse guide rail 112, a telescopic cylinder 113, a sliding member 114, and a tube assembly 115.

Although some embodiments of the present application are described based on a suspended X-ray imaging system, the embodiments of the present application are not limited thereto.

For ease of description, in the present application, the x-axis, y-axis, and z-axis are defined as the x-axis and y-axis being located in the horizontal plane and perpendicular to one another, and the z-axis being perpendicular to the horizontal plane. Specifically, the direction in which the longitudinal guide rail 111 is located is defined as the x-axis, the direction in which the transverse guide rail 112 is located is defined as the y-axis direction, and the direction of extension of the telescopic cylinder 113 is defined as the z-axis direction, and the z-axis direction is the vertical direction.

The longitudinal guide rail 111 and the transverse guide rail 112 are perpendicularly arranged, the longitudinal guide rail 111 being mounted on a ceiling and the transverse guide rail 112 being mounted on the longitudinal guide rail 111. The telescopic cylinder 113 is configured to carry the tube assembly 115.

The sliding member 114 is provided between the transverse guide rail 112 and the telescopic cylinder 113. The sliding member 114 may include components such as a rotating shaft, a motor, and a reel. The motor can drive the reel to rotate around the rotating shaft, which in turn drives the telescopic cylinder 113 to move along the z-axis and/or slide relative to the transverse guide rail. The sliding member 114 is capable of sliding relative to the transverse guide rail 112, i.e., the sliding member 114 is capable of driving the telescopic cylinder 113 and/or the tube assembly 115 to move in the y-axis direction. Further, the transverse guide rail 112 can slide relative to the longitudinal guide rail 111, which in turn drives the telescopic cylinder 113 and/or the tube assembly 115 to move in the x-axis direction.

The telescopic cylinder 113 includes a plurality of cylinders having different inner diameters, and the plurality of cylinders can be sleeved, sequentially from bottom to top, in the cylinder located thereabove, thereby achieving telescoping, and the telescopic cylinder 113 can be telescopic (or movable) in the vertical direction, i.e., the telescopic cylinder 113 can drive the tube assembly to move along the z-axis direction. The lower end of the telescopic cylinder 113 is further provided with a rotating part, and the rotating part can drive the tube assembly 115 to rotate.

The tube assembly 115 includes an X-ray tube, and the X-ray tube may produce X-rays and project the X-rays to an intended region of interest (ROI) of a patient. Specifically, the X-ray tube may be positioned adjacent to a beam limiter, the beam limiter being used to align the X-rays with the intended region of interest of the patient. At least part of the X-rays may be attenuated through the patient, and may be incident on a detector 121/131. In addition, not shown in the drawings, the X-ray imaging system may further include a positionally flexible hand-held detector for imaging some joints or infants.

The suspension apparatus 110 further includes the beam limiter 117, which is usually mounted below the X-ray tube, and the X-rays emitted by the X-ray tube irradiate on the body of a subject through an opening of the beam limiter 117. An irradiation range of the X-rays, namely the region size of an exposure field of view (FOV), depends on the size of the opening of the beam limiter 117. The positions of the X-ray tube and beam limiter 117 in the transverse direction determine the position of the exposure FOV on the body of the subject. It is well known that X-rays are harmful to the human body, so it is necessary to control the X-rays so that the X-rays only irradiate the site of the subject that needs to be examined, namely, the region of interest (ROI).

The suspension apparatus 110 further includes a tube console 116, the tube console 116 being mounted on the tube assembly. The tube console 116 includes user interfaces such as a display screen and a control button, used to perform preparation work before image capture, such as patient selection, protocol selection, positioning, etc.

The movement of the suspension apparatus 110 includes the movement of the tube assembly along the x-axis, y-axis, and z-axis, as well as the rotation of the tube assembly in the horizontal plane (the axis of rotation is parallel to or overlaps with the z-axis) and in the vertical plane (the axis of rotation is parallel to the y-axis). In the above motion, a motor is usually used to drive a rotating shaft which in turn drives corresponding components to rotate in order to achieve the corresponding movement or rotation, and the corresponding control components are generally mounted in the sliding member 114. An X-ray imaging unit further includes a motion control unit (not shown in the figure), and the motion control unit can control the described motion of the suspension apparatus 110. Furthermore, the motion control unit can receive a control signal to control a corresponding component to carry out motion correspondingly.

The wall stand apparatus 120 includes a first detector assembly 121, a wall stand (for example, a chest radiography stand) 122, and a connecting portion 123. The connecting portion 123 includes a support arm that is vertically connected in the height direction of the wall stand 122 and a rotating bracket that is mounted on the support arm, and the first detector assembly 121 is mounted on the rotating bracket. The wall stand apparatus 120 further includes a detector driving apparatus that is arranged between the rotating bracket and the first detector assembly 121, which is driven by the detector driving apparatus to move in a direction parallel to the height direction of the wall stand 122 in the plane held by the rotating bracket, and the first detector assembly 121 can further be rotated relative to the support arm to form an angle with the wall stand. The first detector assembly 121 has a plate-like structure, the orientation of which is variable, facilitating an X-ray incident surface to become vertical or horizontal depending on the incident direction of the X-rays.

In some embodiments, the detector in the embodiments of the present application may include the first detector assembly 121 of the wall stand apparatus 120, wherein a housing of the detector is a housing for housing internal components of the detector, for example, a housing for housing internal components of the first detector assembly 121. The support assembly that supports the detector may include the rotating bracket of the wall stand apparatus 120 for supporting the first detector assembly 121.

The examination table apparatus 130 includes a second detector assembly 131, a table platform 132, and a base 133, the second detector assembly 131 being located below the table platform 132, and the base 133 supporting the second detector assembly 131 and the table platform 132.

In some embodiments, the detector in the embodiments of the present application may include the second detector assembly 131 of the examination table apparatus 130, wherein the housing of the detector is a housing for housing internal components of the detector, e.g., a housing for housing internal components of the second detector assembly 131. The support assembly that supports the detector may include the base 133 of the examination table apparatus 130 for supporting the second detector assembly 131.

The selection or use of the first detector assembly 121 and the second detector assembly 131 may be determined on the basis of an image capture site of a patient and/or an image capture protocol, or may be determined on the basis of the position of the subject that is obtained by the capturing of a camera, to perform an image capture examination in supine or upright positions.

FIG. 1 only shows an example diagram of a wall stand and an examination table, and it should be understood by those skilled in the art that wall stands and/or examination tables of any form or arrangement can be selected, or only the wall stand can be mounted, and the wall stand and/or examination table is not intended to limit the overall solution of the present application.

In some embodiments, the control apparatus 150 may include a source controller and a detector controller. The source controller is used to command an X-ray source to emit X-rays for image exposure. The detector controller is configured to select a suitable detector among a plurality of detectors, and to coordinate the control of various detector functions, such as automatically selecting a corresponding detector based on the position or posture of the subject. Alternatively, the detector controller may perform various signal processing and filtering functions, specifically, for initial adjustment of a dynamic range, interleaving of digital image data, and the like. In some embodiments, the control apparatus may provide power and timing signals for controlling the operation of the X-ray source and the detector.

In some embodiments, the control apparatus may further be configured to use a digitized signal to reconstruct one or more required images and/or determine useful diagnostic information corresponding to the patient, wherein the control apparatus may include one or more dedicated processors, graphics processing units, digital signal processors, microcomputers, microcontrollers, application-specific integrated circuits (ASICs), field-programmable gate arrays (FPGAs), or other appropriate processing apparatuses.

Certainly, the medical imaging system may further include other numbers, configurations, or forms of control apparatuses, for example, the control apparatus may be local (for example, co-located with one or more medical imaging systems 100, such as within the same facility and/or the same local network). In other implementations, the control apparatus may be remote, and thus only accessible through a remote connection (for example, via the Internet or other available remote access technologies). In a specific implementation, the control apparatus may also be configured in a cloud-like means, and may be accessed and/or used in a means that is substantially similar to the means by which other cloud-based systems are accessed and used.

The system 100 further includes a storage apparatus (not shown in the figure). A processor may store the digitized signal in a memory. For example, the memory may include a hard disk drive, a floppy disk drive, a CD-read/write drive, a digital versatile disc (DVD) drive, a flash drive, and/or a solid-state memory. The memory may alternatively be integrated together with the processor to effectively use the footprint and/or meet expected imaging requirements.

The system 100 further includes an input apparatus 160. The input apparatus 160 may include a specific form of operator interface, such as a keyboard, a mouse, a voice-activated control apparatus, a touchscreen (which may also be used as a display apparatus described later), a trackball, or any other suitable input device. An operator may input an operation signal/control signal to the control apparatus by using the input device.

The system 100 further includes a display apparatus 151 (such as a touchscreen or a display screen). The display apparatus 151 may be configured to display an operation interface such as a list of subjects, the positioning or exposure settings of the subjects, and images of the subjects.

In some embodiments, the medical imaging system may further include an image capture apparatus 140. The subject may be captured by using the image capture apparatus to obtain a captured image including the subject, for example, a still image or a series of image frames in a dynamic real-time video stream, to perform positioning assisting, exposure setting, and the like. The image capture apparatus may be mounted on the suspension apparatus, for example mounted on a side edge of the beam limiter 117, and the like, but the embodiments of the present application are not limited thereto.

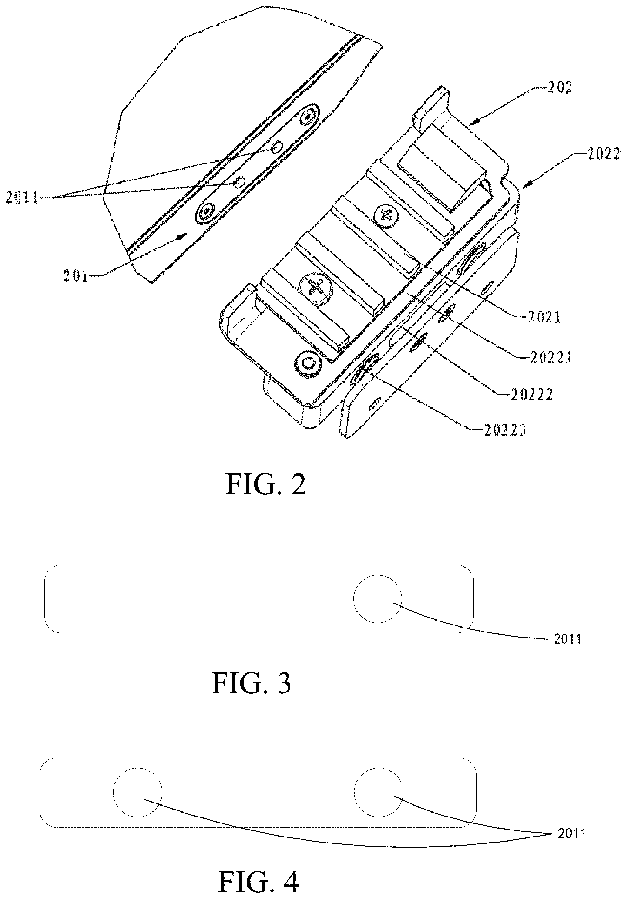

FIG. 2 is a schematic diagram of a detector identification apparatus according to embodiments of the present application. As shown in FIG. 2, the detector identification apparatus 200 includes a marking module 201 and an identification module 202. The marking module 201 is associated with a detector and includes at least one magnetic block 2011. The identification module 202 senses the magnetic block 2011, and generates marking information of the detector according to a sensing result.

According to the above embodiment, the medical imaging system can acquire the marking information of the detector, and, even in a case where there is more than one detector, can identify different detectors, so that an operation process can be simplified, and erroneous operations can be avoided. Furthermore, the detector is marked by means of the magnetic block, which can save costs, is easy to implement, and ensures the reliability of the marking information.

In some embodiments, the medical imaging system may include one or more detectors. For example, the medical imaging system shown in FIG. 1 may include two detectors. The detector may be a wireless communication detector or a wired communication detector, which is not particularly limited in the present application.

In some embodiments, the marking module 201 may be associated with the detector in various ways. For example, the marking module 201 may be disposed on the detector, or the marking module 201 may be disposed on a support assembly that supports the detector.

In some embodiments, when the marking module 201 is disposed on the detector, the identification module 202 may be disposed on the support assembly that supports the detector. Using the medical imaging system shown in FIG. 1 as an example, the marking module 201 may be disposed on the first detector assembly 121, and the identification module 202 may be disposed on the rotating bracket for supporting the first detector assembly 121; alternatively, the marking module 201 may be disposed on the second detector assembly 131, and the identification module 202 may be disposed on the base 133 for supporting the second detector assembly 131.

In some embodiments, when the marking module 201 is disposed on the support assembly that supports the detector, the identification module 202 may be disposed on the detector. Using the medical imaging system shown in FIG. 1 as an example, the marking module 201 may be disposed on the rotating bracket for supporting the first detector assembly 121, and the identification module 202 may be disposed on the first detector assembly 121; alternatively, the marking module 201 may be disposed on the base 133 for supporting the second detector assembly 131, and the identification module 202 may be disposed on the second detector assembly 131.

Hereinafter, using the marking module 201 being disposed on the detector as an example, an exemplary description will be performed for the apparatus according to embodiments of the present application. It will be appreciated that the following description applies equally to the case where the marking module 201 is disposed on an assembly that supports the detector.

In some embodiments, the magnetic block 2011 may be made of various magnetic materials. For example, the magnetic block 2011 is made of magnetic steel.

In some embodiments, the magnetic block 2011 may be disposed on the detector in a preset pattern. The preset patterns of the magnetic blocks 2011 of different detectors may be different. Since different patterns of the magnetic blocks 2011 correspond to different sensing results, different marking information can be generated to distinguish between different detectors.

In some embodiments, the marking information may be a variety of information capable of distinguishing between different detectors. That is, different detectors have different marking information.

The marking information may be represented in various ways. For example, the marking information may be represented by a magnetic sensing signal, e.g., a signal outputted by a magnetic sensor; alternatively, the marking information may also be represented by a signal obtained by performing processing such as filtering on the magnetic sensing signal, e.g., may be represented by high and low levels.

In some embodiments, the preset pattern of the magnetic block 2011 may include at least one of number information, position information, or size information of the magnetic block 2011. For example, at least one of the number, position, or size of the magnetic block 2011 may be different for different detectors. The present application is not limited thereto, and the preset pattern may also include other information of the magnetic block as long as different sensing results or marking information can be generated.

For example, different numbers of magnetic blocks 2011 may be provided for different detectors. FIGS. 3 and 4 are schematic diagrams of the preset pattern according to embodiments of the present application. Using two detectors as an example, as shown in FIG. 3, one magnetic block 2011 may be provided on one detector. As shown in FIG. 4, two magnetic blocks 2011 may be provided on the other detector. When the identification module 202 senses the magnetic blocks 2011 of the two detectors, different sensing results can be generated, and thus different marking information can be generated.

For example, as shown in FIG. 3, the preset pattern includes one magnetic block 2011, and the marking information (detector ID) of the detector corresponding to the preset pattern may be 10 or 01. As shown in FIG. 4, the preset pattern includes two magnetic blocks 2011, and the marking information (detector ID) of the detector corresponding to the preset pattern may be 11.

The present application is not limited thereto, and the magnetic block 2011 may also be configured in alternative quantities. For example, one detector is not provided with any magnetic blocks, while another detector is provided with one or more magnetic blocks.

For another example, the magnetic blocks 2011 may be disposed at different positions for different detectors. Using two detectors as an example, on one detector, the magnetic block may be provided at a first preset position, and on the other detector, the magnetic block may be provided at a second preset position different from the first preset position. When the identification module 202 senses the magnetic blocks of the two detectors, different sensing results can be generated, and thus different marking information is generated.

For another example, different sizes of magnetic blocks 2011 may be provided for different detectors. Using two detectors as an example, a magnetic block having a small size may be provided on one detector, and a magnetic block having a large size may be provided on the other detector. When the identification module 202 senses the magnetic blocks of the two detectors, the sensing intensity of the small magnetic block is less than that of the large magnetic block, so that different sensing results can be generated, and thus different marking information can be generated.

An exemplary description on the structure of the marking module 201 will be performed below with reference to the drawings.

In some embodiments, the marking module 201 may be connected to a housing of the detector. Arrangement of the marking module 201 on the housing of the detector facilitates the identification module 202 in sensing the magnetic block 2011 of the marking module 201, so that the reliability of the sensing result can be ensured, and thus the reliability of the marking information can be ensured.

In some embodiments, the marking module 201 may be connected to the housing of the detector in various ways. For example, the housing of the detector may be provided with a recess recessed toward the inside of the detector, and the marking module 201 may be disposed in the recess. Thus, when the detector is mounted in the support assembly for supporting the detector, interference of the marking module 201 with the support assembly can be avoided, and miniaturization of the medical imaging system can be facilitated.

In some embodiments, the recess self-contained in the housing of the detector may be utilized to accommodate the marking module 201. For example, the recess may accommodate an interface, such as a Type-C interface, for operations such as initial registration of the detector. Since the interface will no longer be used after the initial registration or the like of the detector has been completed, the interface may be removed from the detector and the marking module 201 may be accommodated in the recess that originally accommodated the interface. As a result, changes to the original structure of the detector can be reduced, and implementation is facilitated.

The present application is not limited thereto, and the marking module 201 may also be disposed on other structures of the housing of the detector, or the marking module 201 may be disposed inside the detector as long as it can be accurately sensed by the identification module 202.

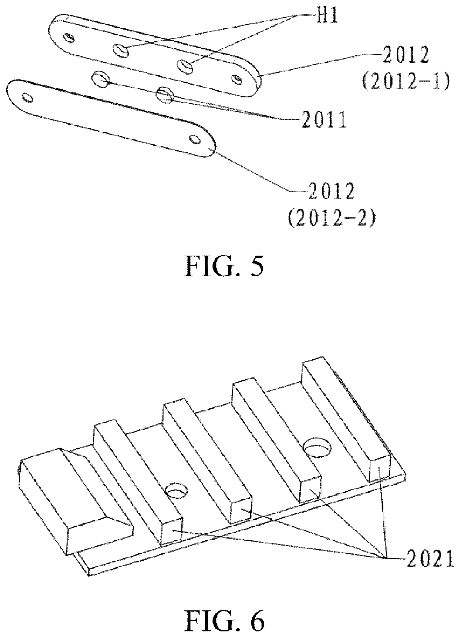

FIG. 5 is a schematic diagram of the marking module 201 according to embodiments of the present application. As shown in FIG. 5, the marking module 201 may further include an accommodating assembly 2012. The accommodating assembly 2012 is connected to the housing of the detector for accommodating the magnetic block 2011. Accommodating the magnetic block 2011 by means of the accommodating assembly 2012 can prevent the magnetic block 2011 from falling out or generating undesired displacement relative to the detector, thereby ensuring the reliability of the sensing results and thus ensuring the reliability of the marking information.

In some embodiments, as shown in FIG. 5, the accommodating assembly 2012 may include: a positioning member 2012-1 and a cover plate 2012-2. The positioning member 2012-1 may be connected to the housing of the detector, and has a positioning hole H1 formed on a side surface away from the housing of the detector, the positioning hole being configured to accommodate the magnetic block. The cover plate 2012-2 may be connected to the positioning member 2012-1 in such a manner as to clamp the magnetic block 2011. By means of the above sandwich-layered structure (also referred to as sandwich structure), the magnetic block 2011 can be securely fixed at a preset position, thereby preventing displacement of the magnetic block 2011 relative to the housing of the detector.

In some embodiments, as shown in FIG. 5, in the positioning member 2012-1, the shape and/or number of the positioning hole H1 matches the shape and/or number of the magnetic block 2011.

For example, the inner diameter of the positioning hole may be slightly greater than the outer diameter of the magnetic block 2011, thereby facilitating mounting of the magnetic block 2011. The magnetic block 2011 and the positioning hole H1 may be substantially circular in shape, such a design facilitating mounting of the magnetic block 2011. The present application is not limited thereto, and the magnetic block 2011 and the positioning hole may be configured in other shapes.

For another example, the number of positioning holes H1 may be equal to or greater than the number of magnetic blocks 2011.

In some embodiments, as shown in FIG. 5, the positioning hole H1 may be a bottomed hole, i.e., a blind hole. The present invention is not limited thereto, and the positioning hole may be a through hole.

In some embodiments, as shown in FIG. 5, the positioning hole H1 is formed in the positioning member 2012-1. The present application is not limited thereto, and the positioning hole H1 may also be formed on a surface of the cover plate 2012-2 close to the detector.

In some embodiments, the accommodating assembly 2012 may be connected to the housing of the detector in various ways. As shown in FIG. 5, a through hole may be formed in the positioning member 2012-1 and the cover plate 2012-2 of the accommodating assembly 2012, and a fixing component such as a screw may be inserted through the through hole to be connected to the housing of the detector. The present application is not limited thereto, and the accommodating assembly 2012 may be connected to the housing by an adhesive connection, a snap connection, or a rivet connection, or the like.

In addition, the accommodating assembly 2012 may also be another structure. For example, the accommodating assembly 2012 may include a positioning member only, a positioning hole is formed in a side surface of the positioning member close to the detector, and the positioning member is connected to the housing of the detector and clamps the magnetic block together with the housing of the detector.

The above is merely an exemplary description of the marking module 201, wherein the marking module 201 may be another structure, which is not particularly limited in the present application.

An exemplary description on the structure of the identification module 202 is performed below with reference to the drawings.

In some embodiments, the identification module 202 may be connected to the support assembly that supports the detector. The support assembly may be a variety of components capable of supporting the detector, such as the aforementioned rotating bracket or base 133.

In some embodiments, as shown in FIG. 2, the identification module 202 may include at least one magnetic sensor 2021. The magnetic sensor 2021 is disposed opposite to the magnetic block 2011, and the distance between the magnetic sensor 2021 and the magnetic block 2011 is less than a first preset distance. By disposing the magnetic sensor 2021 opposite to the magnetic block 2011 and allowing the distance between the magnetic sensor 2021 and the magnetic block 2011 that are disposed opposite to each other to be less than the first preset distance, the reliability of the sensing result can be ensured, and thus the reliability of the marking information can be ensured.

The first preset distance may be related to the magnetic field strength of the magnetic block 2011 and/or the sensing capacity of the magnetic sensor 2021.

FIG. 6 is a schematic diagram of the magnetic sensor according to embodiments of the present application. As shown in FIG. 6, the identification module 202 includes at least one magnetic sensor 2021. One magnetic sensor 2021 may be configured to sense one magnetic block 2011 at a position opposite to the magnetic sensor. Typically, the number of magnetic sensors 2021 corresponds to the number of coded bits of the magnetic block 2011. The present application is not limited thereto, and the number of magnetic sensors 2021 may also be greater than the number of coded bits of the magnetic block 2011. In this case, the marking information may be determined using the sensing result of some of the magnetic sensors 2021.

In some embodiments, a plurality of magnetic sensors 2021 may be spaced apart by a second preset distance, and thus, interference with the sensing results caused by other magnetic blocks 2011 can be prevented.

Similar to the first preset distance, the second preset distance may also be related to the magnetic field strength of the magnetic block 2011 and/or the sensing capacity of the magnetic sensors 2021.

In some embodiments, as shown in FIG. 2, the identification module 202 may further include a mounting assembly 2022. The mounting assembly 2022 may be connected to the magnetic sensor 2021 and the support assembly (not shown) that supports the detector, and mount the magnetic sensor 2021 on the support assembly in such a manner as to float in a first direction, wherein the first direction is, for example, a direction of the magnetic sensor 2021 opposite to the magnetic block 2011, which may also be referred to as an opposite direction.

By mounting the magnetic sensor 2021 in a floating manner by the mounting assembly 2022, it can be ensured that a clearance between the magnetic sensor 2021 and the magnetic block 2011 is less than the first preset distance, so that the reliability of the sensing result can be ensured, and thus the reliability of the marking information can be ensured.

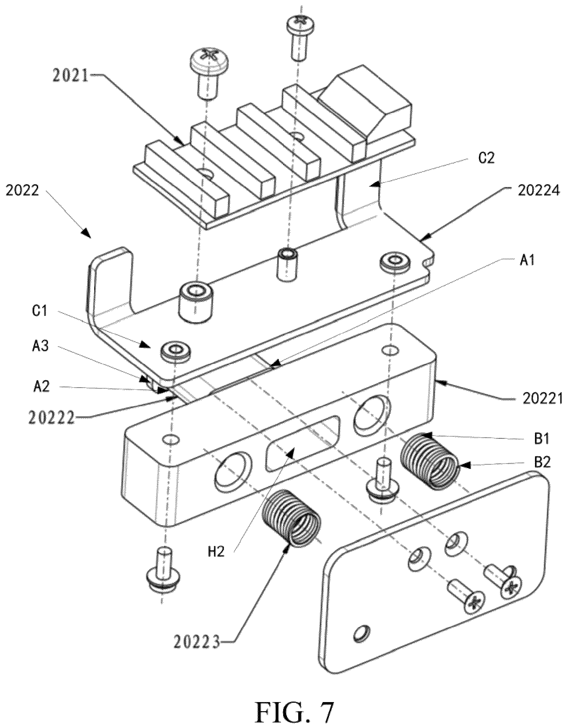

FIG. 7 is a schematic diagram of the mounting assembly according to embodiments of the present application. In some embodiments, as shown in FIGS. 2 and 7, the mounting assembly 2022 may include: a body member 20221, a guide member 20222, and an elastic member 20223. The body member 20221 is connected to the magnetic sensor 2021.

As shown in FIG. 7, a first end A1 of the guide member 20222 is connected to the support assembly (not shown), and the body member 20221 drives the magnetic sensor 2021 to move along the guide member 20222. A first end B1 of the elastic member 20223 is connected to the body member 20221, and a second end B2 of the elastic member 20223 is connected to the support assembly (not shown).

By disposing the elastic member 20223 in the mounting assembly 2022, when the marking module 201 abuts against the identification module 202, the position of the identification module 201 can be adjusted according to the position of the marking module 202, so that the marking module 201 and the identification module 202 can be kept in a state of abutment, thereby ensuring that the distance between the magnetic sensor 2021 and the magnetic block 2011, which are oppositely provided, is less than the first preset distance.

In some embodiments, the body member 20221 and the magnetic sensor 2021 may be connected in various ways. For example, the magnetic sensor 2021 may be connected to the body member 20221 by a threaded connection, an adhesive connection, a snap connection, or a rivet connection, or the like.

In some embodiments, the guide member 20222 may be various structures having a guiding function. For example, the guide member 20222 may be a guide rod, a guide rail, or the like. For example, as shown in FIG. 7, the guide member 20222 may have a substantially columnar shape, an extension direction thereof being the first direction. The body member 20221 has a hole portion H2 formed therein, at least a portion of the guide member 20222 being located in the hole portion H2. Thus, the body member 20221 can be moved in the first direction along the guide member 20222.

In some embodiments, to prevent the body member 20221 from slipping off the guide member 20222, a limiting portion A3 may be formed at a second end A2 of the guide member 20222. For example, when the body member 20221 is located at a first position, an end surface of the limiting portion A3 facing the support assembly abuts against an end surface of the body member 20221 facing the detector. As a result, the body member 20221 cannot slide to a position further away from the support assembly than the first position, so that the body member 20221 can be prevented from falling out.

The present application is not limited thereto, and the guide member 20222 may also not be provided with the limiting portion A3, and the body member 20221 may be prevented from falling out by means of another member or structure. For example, the first end B1 of the elastic member 20223 may be fixedly connected to the body member 20221 and the second end B2 of the elastic member 20223 may be fixedly connected to the support assembly, which may also prevent the body member 20221 from falling out.

In some embodiments, the elastic member 20223 may be a variety of elastic structures, for example, the elastic member 20223 may be a spring or the like. As previously described, both ends of the elastic member 20223 may be fixedly connected to the body member 20221 and the support assembly, respectively. The present application is not limited thereto, and both ends of the elastic member 20223 may also abut against the body member 20221 and the support assembly, respectively, or one end may be fixedly connected, and the other end may be abutting, or the like. As a result, a mounting operation of the elastic member 20223 can be simplified.

In some embodiments, one or more guide members 20222 may be provided, and one or more elastic members 20223 may be provided.

For example, the guide member 20222 may be located at a position coinciding with a central axis of the body member 20221, a plurality of elastic members 20223 may be provided, and the elastic members may be disposed symmetrically relative to the body member 20221. As a result, the body member 20221 can be prevented from tilting, thereby ensuring that the body member 20221 moves stably along the guide member 20222.

As shown in FIG. 7, the elastic member 20223 and the guide member 20222 may be spatially separated and disposed at different positions. The present application is not limited thereto, and the elastic member 20223 and the guide member 20222 may be arranged in other manners. For example, the elastic member 20223 may be sleeved on the outer peripheral side of the guide member 20222, the first end B1 of the elastic member 20223 may be connected to the body member 20221, and the other end B2 may be connected to the support assembly, or the other end B2 may be connected to the guide member 20222, etc.

In some embodiments, as shown in FIG. 7, the mounting assembly 2022 may further include a blocking member 20224. The blocking member 20224 may be connected to the body member 20221, an end surface of the blocking member 20224 facing the detector may be located at a position flush with an end surface of the magnetic sensor 2021 facing the detector, or an end surface of the blocking member 20224 facing the detector may be located at a position closer to the detector than an end surface of the magnetic sensor 2021 facing the detector. By providing the blocking member 20224, when the marking module 201 abuts against the identification module 202, it is possible to prevent the magnetic sensor 2021 from being damaged due to an impact on the magnetic sensor 2021.

As shown in FIG. 7, the blocking member 20224 may include a connecting portion C1 connected to the body member 20221 and shielding pieces C2 connected to an edge of the connecting portion C1 close to the detector and substantially perpendicular to the connecting portion C1. The shielding pieces C2 are disposed on both sides of the magnetic sensor 2021, respectively, so that the magnetic sensor 2021 can be reliably protected from the impact of the detector. A surface of each shielding piece C2 close to the detector may be a substantially flat surface, so that the detector can be stably supported, and thus the body member 20221 can be prevented from tilting.

The above is merely an exemplary description of the identification module 202, wherein the identification module 202 may be another structure, which is not particularly limited in the present application.

It is worth noting that only the individual components or modules relevant to this embodiment have been described above, but the present application is not limited thereto. The detector identification apparatus may further include other components or modules, the specifics of which can be found in the related art.

According to the above embodiments, the detector identification apparatus includes the marking module and the identification module, wherein the marking module is associated with the detector and includes at least one magnetic block; and the identification module senses the magnetic block, and generates the marking information of the detector according to the sensing result. Thus, the medical imaging system can acquire the marking information of the detector, and, even in a case where there is more than one detector, can identify different detectors, so that the operation process can be simplified, and erroneous operations can be avoided. Furthermore, the detector is marked by means of the magnetic block, which can save costs, is easy to implement, and ensures the reliability of the marking information.

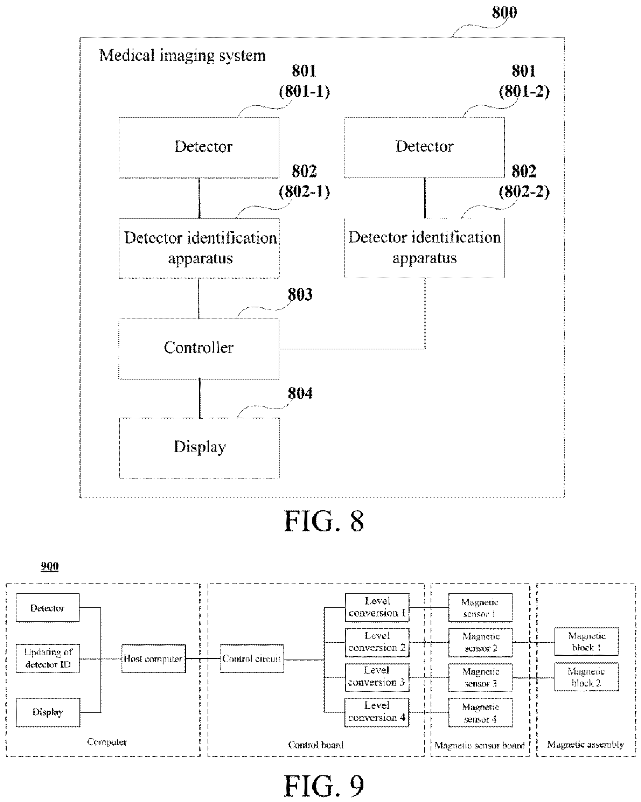

Embodiments of the present application further provide a medical imaging system. FIG. 8 is another schematic diagram of the medical imaging system according to the embodiments of the present application. As shown in FIG. 8, the system 800 includes: a detector 801, a detector identification apparatus 802, and a controller 803.

The detector identification apparatus 802 is configured to generate marking information of the detector 801, and for the implementation of the detector identification apparatus, reference may be made to the foregoing embodiments, which will not be repeated here. The controller 803 generates, according to the marking information of the detector 801 generated by the detector identification apparatus 802, indication information related to the detector 801.

In some embodiments, the indication information may include various information related to the detector 801. For example, the indication information may be used to indicate whether a corresponding detector is mounted in the medical imaging system, an operation state of the corresponding detector, or the like. Alternatively, the indication information may be used to instruct a display described later to display a mounting position of the detector 801, for example, the detector 801 is mounted in a wall stand apparatus and/or an examination table apparatus.

In some embodiments, in the medical imaging system 800, the detector 801 may include one or more detectors, and the detector identification apparatus 802 may include one or more detector identification apparatuses. As shown in FIG. 8, the detector 801 may include detectors 801-1 and 801-2. The detector identification apparatus 802 may include detector identification apparatuses 802-1 and 802-2. The detector identification apparatus 802-1 is configured to generate first marking information of the detector 801-1, the detector identification apparatus 802-2 is configured to generate second marking information of the detector 801-2, and the controller 803 may receive the first marking information and the second marking information and thus generate corresponding indication information.

In some embodiments, the controller 803 may be configured separately from a controller of the medical imaging system. For example, the controller 803 is configured as a chip or the like connected to the controller of the medical imaging system, and the two controllers may control each other. Alternatively, functions of the controller may also be integrated into the controller of the medical imaging system. Embodiments of the present application are not limited thereto.

In some examples, the controller 803 includes a computer processor and a storage medium. Recorded on the storage medium is a program for predetermined data processing to be executed by the computer processor. The storage medium may include, for example, a ROM, a floppy disk, a hard disk, an optical disk, a magneto-optical disk, a CD-ROM, or a non-volatile memory card.

In some embodiments, as shown in FIG. 8, the system 800 may further include a display 804. The display 804 displays the position information of the detector 801 based on the instruction information.

For example, the display 804 may display the position of a detector currently mounted in the medical imaging system. For example, a detector mounted in the wall stand apparatus and/or the examination table apparatus in the medical imaging system is displayed. The present application is not limited thereto, and the display may also display other information of the detector.

FIG. 9 is another schematic diagram of a medical imaging system according to embodiments of the present application. An exemplary description of the concrete implementation of the medical imaging system is provided below with reference to FIG. 9. As shown in FIG. 9, in a medical imaging system 900, a magnetic sensor in a magnetic sensor board that is connected to a control board on which level conversion circuits and a control circuit (MCU/FPGA) are mounted senses a magnetic block associated with a detector. A detector identifier (ID) can be identified, and the position of the detector can be displayed on a display by performing logic control via a host computer.

As shown in FIG. 9, the number of coded bits of magnetic blocks is 2, and four magnetic sensors are disposed in the magnetic sensor board, wherein marking information can be generated using sensing results of two magnetic sensors (e.g., magnetic sensor 2 and magnetic sensor 3) among the four magnetic sensors. Alternatively, only two magnetic sensors may be disposed in the magnetic sensor board.

When a signal in a magnetic sensor is triggered (i.e., the magnetic sensor detects a corresponding magnetic block), the control board monitors the signal of the magnetic sensor in real time and reports the signal that includes detector ID information to the host computer through a preset protocol.

Upon receiving the signal that includes the detector ID information, the host computer monitors the signal in real time, and can update the detector ID information in real time when the signal changes. The host computer controls the display to display the position of the detector corresponding to the detector ID in the medical imaging system. The information will help support a user to optimize a working process.

In some embodiments, each level conversion circuit may be any of a variety of circuits, for example, the level conversion circuit may include a Schmitt trigger or the like.

The medical imaging system includes, but is not limited to, a computed tomography (CT) system, a magnetic resonance imaging (MRI) system, a C-arm imaging system, a positron emission computed tomography (PET) system, a single photon emission computed tomography (SPECT) system, an ultrasound system, an X-ray imaging system, or any other suitable medical imaging system.

Although some embodiments of the present application are described on the basis of the suspended X-ray imaging system shown in FIG. 1, the embodiments of the present application are not limited thereto. For example, the medical imaging system may also be a floor-standing X-ray imaging system, a mobile X-ray imaging system, or the like. Examples are not listed herein one by one again.

The medical imaging system may further include other structural components not shown in the drawings. The embodiments of the present application are not limited thereto. For example, reference may be made to FIG. 1, and specific reference can be made to the related art, which will not be described here again.

According to the above embodiments, a detector identification apparatus is provided in a medical imaging system, the detector identification apparatus including a marking module and an identification module, wherein the marking module is associated with a detector and includes at least one magnetic block; and the identification module senses the magnetic block, and generates marking information of the detector according to the sensing result. Thus, the medical imaging system can acquire the marking information of the detector, and, even in a case where there is more than one detector, can identify different detectors, so that an operation process can be simplified, and erroneous operations can be avoided. Furthermore, the detector is marked by means of the magnetic block, which can save costs, is easy to implement, and ensures the reliability of the marking information.

The above embodiments merely provide illustrative descriptions of the embodiments of the present application. However, the present application is not limited thereto, and appropriate variations may be made on the basis of the above embodiments. For example, each of the embodiments described above may be used independently, or one or more among the above embodiments may be combined.

The present application is described above with reference to specific embodiments. However, it should be clear to those skilled in the art that the foregoing description is merely illustrative and is not intended to limit the scope of protection of the present application. Various variations and modifications may be made by those skilled in the art according to the spirit and principle of the present application, and these variations and modifications also fall within the scope of the present application. As used herein, the term "exemplary" means serving as a non-limiting example, instance, or illustration. As used herein, the term "for example" introduces a list of one or more non-limiting examples, instances, or illustrations.

Preferred embodiments of the present application are described above with reference to the accompanying drawings. Many features and advantages of the embodiments are clear according to the detailed description. Therefore, the appended claims are intended to cover all these features and advantages that fall within the true spirit and scope of these embodiments. In addition, as many modifications and changes could be easily conceived of by those skilled in the art, the embodiments of the present application are not limited to the illustrated and described precise structures and operations, but can encompass all appropriate modifications, changes, and equivalents that fall within the scope of the embodiments.

## Claims

1. A detector identification apparatus, **characterized by** comprising:
a marking module associated with a detector and comprising at least one magnetic block; and
an identification module, the identification module sensing the magnetic block and generating marking information of the detector according to a sensing result.

2. The apparatus according to claim 1, wherein
the marking module is disposed on the detector, or the marking module is disposed on a support assembly supporting the detector.

3. The apparatus according to claim 1, wherein
the magnetic block is disposed on the detector in a preset pattern comprising at least one of number information, position information, or size information of the magnetic block.

4. The apparatus according to claim 3, wherein
the preset patterns of the magnetic blocks disposed on different detectors are different.

5. The apparatus according to claim 1, wherein
the marking module is connected to a housing of the detector; and
the identification module is connected to a support assembly supporting the detector.

6. The apparatus according to claim 1, wherein the marking module further comprises:
an accommodating assembly configured to accommodate the magnetic block, the accommodating assembly being connected to a housing of the detector.

7. The apparatus according to claim 6, wherein the accommodating assembly comprises:
a positioning member connected to the housing of the detector and having a positioning hole formed on a side surface away from the housing of the detector, the positioning hole being configured to accommodate the magnetic block; and
a cover plate connected to the positioning member in such a manner as to clamp the magnetic block.

8. The apparatus according to claim 1, wherein the identification module comprises:
at least one magnetic sensor disposed opposite to the magnetic block, the distance between the magnetic sensor and the magnetic block being less than a first preset distance.

9. The apparatus according to claim 8, wherein the identification module further comprises:
a mounting assembly connected to the magnetic sensor and a support assembly supporting the detector, and mounting the magnetic sensor on the support assembly in such a manner as to float in a first direction, the first direction being a direction of the magnetic sensor opposite to the magnetic block.

10. The apparatus according to claim 9, wherein the mounting assembly comprises:
a body member connected to the magnetic sensor;
a guide member, a first end of the guide member being connected to the support assembly, and the body member driving the magnetic sensor to move along the guide member; and
an elastic member, a first end of the elastic member being connected to the body member, and a second end of the elastic member being connected to the support assembly or the guide member.

11. The apparatus according to claim 10, wherein
the body member has a hole portion formed therein, at least a portion of the guide member being located in the hole portion, and
the guide member has a limiting portion formed at a second end thereof, and when the body member is located at a first position, an end surface of the limiting portion facing the support assembly abuts against an end surface of the body member facing the detector.

12. The apparatus according to claim 10, wherein
the guide member is located at a position coinciding with a central axis of the body member, and
a plurality of elastic members are provided, and the elastic members are located at positions symmetrical relative to the body member.

13. The apparatus according to claim 10, wherein the mounting assembly further comprises:
a blocking member connected to the body member, an end surface of the blocking member facing the detector being located at a position flush with an end surface of the magnetic sensor facing the detector, or an end surface of the blocking member facing the detector being located at a position closer to the detector than an end surface of the magnetic sensor facing the detector.

14. A medical imaging system, **characterized by** comprising:
a detector,
the detector identification apparatus according to any one of claims 1 to 13; and
a controller, the controller, according to marking information of the detector generated by the detector identification apparatus, generating indication information related to the detector.

15. The system according to claim 14, further comprising:
a display, the display displaying position information of the detector according to the indication information.
